Europäisches Patentamt

**European Patent Office**

**Office européen des brevets**

(19)

(11) Veröffentlichungsnummer: **0 005 243**
A2

(12) **EUROPÄISCHE PATENTANMELDUNG**

(21) Anmeldenummer: **79101263.6**

(22) Anmeldetag: **26.04.79**

(51) Int. Cl.²: **G 01 N 33/16, A 61 K 35/12**

(30) Priorität: **27.04.78 CH 4582/78**

(43) Veröffentlichungstag der Anmeldung: **14.11.79**
**Patentblatt 79/23**

(84) Benannte Vertragsstaaten: **CH DE FR GB IT NL**

(71) Anmelder: **F.Hoffmann-La Roche & Co.**
**Aktiengesellschaft, Abt. VIII-Pt, CH-4002 Basel (CH)**

(72) Erfinder: **Strässle, Rudolf, Dr., Burgstrasse 4, CH-4125**
**Riehen (CH)**
Erfinder: **Zaugg, Hans, Dr., Obere Flühackerstrasse 7,**
**CH-4402 Frenkendorf (CH)**

(74) Vertreter: **Lederer, Franz, Dr. et al, Patentanwälte Dr.**
**Lederer Franz Meyer-Roxlau Reiner F. Lucile-Grahn-**
**Strasse 22, D-8000 München 80 (DE)**

(54) **Kontrollplasma, dessen Herstellung, dessen Verwendung zur Ueberwachung der oralen Antikoagulantientherapie sowie Reagenziengarnitur enthaltend dieses Kontrollplasma.**

(57) Kontrollplasma nicht menschlicher Herkunft zur Überwachung der Antikoagulantientherapie beim Menschen, welches aus dem Blut stabil antikoagulierter Säugetiere gewonnen wird.

EP 0 005 243 A2

0005243

26. April 1979

F. Hoffmann-La Roche & Co. Aktiengesellschaft, Basel, Schweiz

RAN 4094/3

<u>Kontrollplasma, dessen Herstellung, dessen Verwendung zur Ueberwachung der oralen Antikoagulantientherapie sowie Reagenziengarnitur enthaltend dieses Kontrollplasma.</u>

<u>Vorklassifizierungsnummer: CP 78/004121.</u>

Die Anwendung oraler Antikoagulantien, z.B. von Cumarinderivaten, zur Thromboseprophylaxe nach Operationen oder zur Langzeitbehandlung von Infarktpatienten und Patienten mit thrombo-embolischen Erkrankungen, muss bekanntlich sehr genau überwacht werden. Bei Ueberdosierung können Blutungen, bei Unterdosierungen thrombo-embolische Ereignisse eintreten.

Aus diesem Grund ist es notwendig,zum Schutz des Patienten ständig zu kontrollieren,ob die Dosierungen richtig gewählt wurden. Dies erfolgt in der Regel durch Bestimmung der Gerinnungszeit des mit Thromboplastin und Calcium versetzten Plasmas (Thromboplastinzeit) des Patienten nach Methoden, die auf der Bestimmung, welche von Quick, A.J. in The Physiology and Pathology of Hemostasis p. 125, London Klimpton 1951 beschrieben wurde, basieren. Nach einer Operation erfolgt eine derartige Ueberwachung der Therapie zuerst alle Tage, dann in grösseren zeitlichen Abständen.

Klt/13.2.1979

- 2 -                              0005243

Die Dosierung des Antikoagulans ist richtig gewählt worden, wenn die erhaltenen Quickwerte oder das Thromboplastinzeitverhältnis [vergleiche Biggs et al, British Medical Journal (1967) p. 84-88] im sogenannten therapeutischen Bereich liegen. Dieser therapeutische Bereich, in welchem das Risiko sowohl von Blutungen, als auch von Thrombosen minimal ist, kann in Quickwerten (prozentuale Verdünnung eines Mischplasmas von mehreren gesunden Spendern, welche die gleiche Thromboplastinzeit ergibt, wie die zu untersuchende Plasmaprobe) oder in Thromboplastinzeitquotienten (Verhältnis der Thromboplastinzeit des zu untersuchenden Patientenplasmas zur Thromboplastinzeit eines auf gleiche Weise gewonnenen Mischplasmas von mehreren gesunden Spendern) ausgedrückt werden. Sowohl die Quickwerte als auch die Thromboplastinzeitquotienten sind vom eingesetzten Thromboplastin abhängig. In der Regel liegt jedoch der therapeutische Bereich ungefähr bei einem Quickwert zwischen 10-35% oder einem Thromboplastinzeitquotienten zwischen 1.5 und 5.0. Es hat sich jedoch herausgestellt, dass die Richtigkeit und Präzision der Bestimmung des Quickwertes oder des Thromboplastinzeitquotienten nicht nur durch Verwendung eines normalen Kontrollplasmas (Quickwert 70-100%; Thromboplastinzeitquotient z.B. 1.15-1) sondern auch eines Kontrollplasmas mit einem Quickwert bzw. einem Thromboplastinzeitquotienten im therapeutischen Bereich (nachstehend als pathologisches Kontrollplasma bezeichnet), kontrolliert werden muss.

Bisher wurden pathologische Kontrollplasmen meistens durch Adsorption von verschiedenen Gerinnungsfaktoren, insbesondere der Faktoren II, VII, IX und X hergestellt. In einem bevorzugten Verfahren wird menschliches Plasma mit Aluminiumhydroxid gerührt und die Mischung zentrifugiert. Das Sediment enthält die zu entfernenden Gerinnungsfaktoren und der Ueberstand liefert, nach allfälligem Mischen mit normalem nicht adsorbiertem Plasma, pathologisches Kontrollplasma mit einem Quickwert oder einem Thromboplastinzeitquotient im therapeutischen Bereich.

Pathologische Kontrollplasmen, die auf diese Weise hergestellt wurden, befriedigen jedoch nicht völlig, weil ihre Zusammensetzung sich doch sehr wesentlich von derjenigen des Patientenplasmas unterscheidet. Insbesondere fehlen in einem solchen Kontrollplasma die im Patientenplasma vorhandenen PIVKA-Faktoren (PIVKA = Proteins Induced by Vitamin K Absence or Antagonism.) Diese PIVKA-Faktoren, welche von verschiedenen Thromboplastinreagenzien verschieden empfindlich erfasst werden, haben einen wesentlichen Einfluss auf den gemessenen Quickwert oder auf den Thromboplastinzeitquotienten und sollten somit, um eine zuverlässige Kontrolle im therapeutischen Bereich sicherzustellen, im pathologischen Kontrollplasma vorhanden sein.

Infolge der Nachteile der durch Adsorption hergestellten pathologischen Kontrollplasmen ist man z.T. dazu übergegangen, als Kontrollplasma Plasma von stabil eingestellten oral antikoagulierten lebergesunden Patienten einzusetzen. Ein solches Kontrollplasma, welches einen Quickwert oder einen Thromboplastinzeitquotienten im therapeutischen Bereich aufweist,und,unter anderem, die wichtigen PIVKA-Faktoren enthält, besitzt eine dem Patientenplasma weitgehend entsprechende Zusammensetzung und ermöglicht somit die besonders komplexe und störanfällige Thromboplastinzeitbestimmung durch eine wirksamere Qualitätskontrolle abzusichern. Ein wesentlicher Nachteil ist jedoch, dass als Ausgangsmaterial zur Herstellung solcher antikoagulierter pathologischer Kontrollplasmen die Reste von Patientenplasmaproben, wie sie in Kliniklaboratorien anfallen, dienen. Dies ist umständlich, weil die Plasmaproben antikoagulierter Patienten täglich aus verschiedenen Spitälern eingesammelt und tiefgefroren aufbewahrt werden müssen. Im Gegensatz zu normalen Blutspendern, welche im gesunden Zustand freiwillig ihr Blut spenden, wird hier das im Ueberschuss genommene Blut von kranken Menschen eingesetzt. Diese Methode mag im kleineren Massstab vertretbar und durchführbar sein, kann jedoch sowohl aus ethischen als auch aus technischen und logistischen Gründen keineswegs von der gesamten diagnostischen Industrie verwendet werden.

Es wurde nun gefunden, dass durch Antikoagulation von Säugetieren, insbesondere von Schafen, ein pathologisches Kontrollplasma mit einem Quickwert und einem Thromboplastinzeitquotienten im therapeutischen Bereich hergestellt werden kann, das eine dem Patientenplasma analoge Zusammensetzung aufweist und unter anderem die wichtigen PIVKA-Faktoren enthält. Das erfindungsgemässe pathologische Kontrollplasma aus Säugetieren gewährleistet die gleiche Qualität der Kontrolle, wie das antikoagulierte pathologische Kontrollplasma von Patienten, ohne die im vorhergehenden Paragraphen geschilderten Nachteile aufzuweisen.

Genauer gesagt betrifft die vorliegende Erfindung ein Kontrollplasma nicht menschlicher Herkunft zur Ueberwachung der oralen Antikoagulantientherapie beim Menschen, hergestellt aus Blut von stabil eingestellten antikoagulierten Säugetieren.

Es wurde gefunden, dass dieses Kontrollplasma sich von Kontrollplasma aus stabil antikoagulierten Menschen nur verhältnismässig wenig unterscheidet, und insbesondere in funktioneller Hinsicht dem Kontrollplasma stabil antikoagulierter Patienten entspricht. Der wesentliche Unterschied, nämlich die höhere Faktor V-Aktivität (Verhältnis der Faktor V-Aktivität zu derjenigen eines Humanplasmas grösser als 2, insbesondere zwischen 2 und 5 ) des erfindungsgemässen Kontrollplasmas, hat keinen Einfluss auf seine Qualität zur Kontrolle der Thromboplastinzeitbestimmung.

Das erfindungsgemässe Kontrollplasma kann hergestellt werden, indem man Säugetiere mit einem Antikoagulans behandelt, sie mittels Ueberwachung von Quickwert oder Thromboplastinzeitquotienten auf einen stabilen Wert im therapeutischen Bereich einstellt, das Plasma nach Blutentnahme in einem Mittel, das die spontane Blutgerinnung verhindert, durch Zentrifugation gewinnt und dem erhaltenen Plasma einen Puffer zwecks Einstellung eines physiologischen pH-Wertes zusetzt.

Säugetiere, welche sich besonders für die Zwecke der vorliegenden Erfindung eignen,sind Rinder, Pferde, Ziegen, Kaninchen, Schweine, Hunde und Schafe. Besonders bevorzugt sind Schafe.

Die Art des verwendeten Antikoagulans ist nicht kritisch. Vorzugsweise werden jedoch Cumarinderivate wie 3-(1-Phenyl-propyl)-4-hydroxy-cumarin (Phenprocoumon); 3-(α-[4-Nitrophenyl]-β-acethyläthyl)-4-hydroxy-cumarin (Acenocoumarin); 3-(α-Phenyl-β-Acetyläthyl)-4-hydroxy-cumarin (Warfarin sodium); 3,3'-Carbäthoxymethylen-bis-(4-hydroxy-cumarin) (Aethylbiscoumacetat); 3,3'-Methylen-bis-(4-hydroxy-cumarin) (Bishydroxycoumarin) eingesetzt. Besonders bevorzugt ist 3-(1-Phenyl-propyl)-4-hydroxycumarin.

Die Behandlung kann sowohl oral als auch intravenös erfolgen. Eine orale Behandlung ist jedoch bevorzugt. Die Art der Behandlung, insbesondere die eingesetzten Dosen und die Dauer dieser Behandlung hängen unter anderem vom verwendeten Antikoagulans, von der Tierspezies, vom Gewicht und von der Empfinlichkeit des Tieres ab. In der Regel werden jedoch Dosen von ungefähr O.8 mg/kg, mindestens täglich, während wenigstens einem Monat dem Tier verabreicht. Das Tier erhält vorzugsweise während der gesamten Behandlung immer Futter der gleichen Zusammentsetzung.

Zur Ueberwachung des Thromboplastinzeitquotienten oder des Quickwerts werden periodisch, vorzugsweise etwa 2 mal wöchentlich, durch Venenpunktion Blutproben entnommen.

Zur Herstellung des gewünschten Kontrollplasmas wird nach stabiler Einstellung des Quickwertes oder Thromboplastinzeitquotienten im therapeutischen Bereich in angemessenen Zeitabständen (bei Schafen höchstens alle zwei Wochen), in Gegenwart eines Mittels zur Verhinderung der spontanen Blutgerinnung, vorzugsweise durch Venenpunktion, Blut entnommen. Als Mittel zur Verhinderung der spontanen Blutgerinnung

eignen sich Komplexbildner für Calcium-Ionen wie z.B. Trinatriumcitrat, Aethylendiamintetraessigsäure (EDTA) oder Natriumoxalat. Diese Komplexbildner werden in Mengen und Konzentrationen eingesetzt, die bei der Blutentnahme beim Menschen üblich sind. So wird Trinatriumcitrat vorzugsweise in Form einer 0.1 molaren Lösung in einem Verhältnis von 1 zu 9 zum entnommenen Blut eingesetzt.

Aus dem erhaltenen Blut wird auf übliche Art durch Zentrifugation Plasma erhalten. Mit Vorteil werden zur Herstellung des gewünschten Kontrollplasmas mehrere auf diese Weise erhaltene Plasmaportionen gemischt.

Zur Stabilisierung des Kontrollplasmas auf einen physiologischen pH-Wert (7.2 bis 7.5) wird vorzugsweise ein geeigneter Puffer wie z.B. Tris-Puffer, Phosphatpuffer, Imidazolpuffer, oder N-2-Hydroxyäthylpiperazin-N'-aethansulfonsäure (HEPES) zugefügt.

Um sicherzustellen, dass im Plasma die Thrombocyten weitgehend entfernt werden, wird das Plasma vorzugsweise zentrifugiert und allfällig noch vorhandenes Sediment entfernt.

Das erfindungsgemässe Kontrollplasma kann im flüssigen, gefrorenen oder lyophilisierten Zustand vorliegen. Vorzugsweise wird es in lyophilisierter Form aufbewahrt oder in Reagenziengarnituren zur Kontrolle der Thromboplastinzeit verpackt.

Eine solche Reagenziengarnitur kann zusätzlich in weiteren Berhältern das Thromboplastinreagens, und/oder das normale Kontrollplasma und/oder eine Lösung zur Verhinderung der spontanen Blutgerinnung enthalten.

Die Erfindung wird anhand der nachstehenden Beispiele näher erläutert.

Beispiel

Orale Antikoagulation von Schafen

10 Schafe (weiblich, nicht trächtig, mittleres Gewicht ca. 50 kg) werden als Gruppe eingestallt. Die Tiere sind durch Ohrmarken gekennzeichnet und für jedes Tier liegt ein Protokollblatt bereit, auf dem das Tiergewicht vor Behandlungsbeginn sowie alle Manipulationen während der Behandlungsdauer eingetragen werden. An der Futterzusammensetzung wird während der Behandlungsdauer nichts geändert. Phenprocoumon-Kapseln [20 mg 3-(1-Phenyl-propyl-4-hydroxy-cumarin in einer Gelatinsteckkapsel] werden jedem Tier durch eine Schlundröhre eingegeben und zwar morgens und eventuell abends. Die Dosierung erfolgt individuell für jedes Tier. An den ersten vier Tagen werden den Tieren in der Regel absteigende Dosen, z.B. 4 Kapseln am 1., 3 Kapseln am 2., 2 Kapseln am 3. und 1 Kapsel am 4. Tag, verabreicht. Die weitere Dosierung erfolgt individuell aufgrund des bestimmten Quickwertes. Dies hat z.B. bei einem Tier das nachfolgende Dosierungsschema ergeben:

| Behandlungstag | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|
| Anzahl Kapseln pro Tag | 4 | 3 | 2 | 1 | 2 | 2 | 1 | 2 | 2 | 1 | 2 | 2 | 1 | 2 | 2 | 1 | 2 | 2 | 1 | 2 |

| Behandlungstag | 21 | 22 | 23 | 24 | 25 | 26 | 27 | 28 |
|---|---|---|---|---|---|---|---|---|
| Anzahl Kapseln | 2 | 2 | 2 | 2 | 1 | 2 | 2 | 1 |

Die Kontrollblutentnahmen zur Quickwert-Bestimmung werden jeweils vor der täglichen Phenprocoumon-Gabe durchgeführt, indem mit einer Spritze 9 ml Blut/Tier in 1 ml 0.1 M Tri-Natriumcitrat gegeben werden. Die Quickwert-Bestimmung mit Plasma erfolgt zuerst täglich, dann wöchentlich 1-2 mal. Nach ca. 30 Tagen ab Behandlungsbeginn sind die Tiere innerhalb des therapeutischen Bereiches eingestellt.

Herstellung von pathologischem Kontrollplasma

In Blutspendeflaschen 500 ml, silikonisiert, werden 40 ml O.1 M Natriumcitratlösung gegeben. Durch Venenpunktion (V. jugularis) wird sodann das Blut der mit Phenprocoumon behandelten Schafen bis zur 400 ml-Marke in die Flasche einlaufen gelassen. Während dieser Zeit wird durch leichtes Umschwenken der Flasche ständig für eine gute Durchmischung gesorgt. Das aufgefangene Blut wird dann so rasch wie möglich zu Plasma weiterverarbeitet. Die Zentrifugation erfolgt bei 2500 U/Min. (ca. 1600 xg) und 10°C während 30 Min. Das Plasma wird sorgfältig mit silikonisierter Pipette und Propipette abgesaugt und in 250 ml Plastikbecher gegeben. Die Thromboplastinzeit (TPZ) der Einzelplasmen wird bestimmt und es wird dann anhand der Quickwerte je 200 ml Einzelplasma gepoolt. Der gefundene Quickwertbereich des Plasmapools beträgt 20 - 25% für Calcium-Thromboplastin-Roche. Es erfolgt der Zusatz von fester N-2-Hydroxyäthyl-piperazin-N'-äthansulfonsäure (HEPES) bis zur Konzentration von 0.05 M (11.9 g HEPES/1000 ml Plasma). Nach Zentrifugation (15 Min; 7000 xg) bei 10°C in Plastikbechern wird das überstehende Plasma sorgfältig abdekantiert und so rasch wie möglich in 0.5 ml Anteilen in Ampullenfläschchen abgefüllt und lyophilisiert.

## Eigenschaften

a)   Quickwert und Thromboplastinzeitquotient

Der Ampulleninhalt wird im entsprechenden Volumen dest. Wasser gelöst. Die Lösung wird 5 Minuten bei 37°C, dann 10 Minuten bei Raumtemperatur stehengelassen und die Thromboplastinzeitbestimmung (TPZ) mit Calcium-Thromboplastin Roche durchgeführt.

| Ansatz | Ca-Tpl-Roche Quickwert | Thromboplastinzeitquotient |
|--------|------------------------|----------------------------|
| 1 | 25 % | 1.78 |
| 2 | 24.5 % | 1.81 |
| stabil antikoaguliertes Humanplasma | 24 % | 1.7 |

### Faktorengehalt

Die Gerinnungsfaktoren II, V, VII/X und X wurden nach der Methode von Marbet und Winterstein (J.Jürgens/F.K.Beller Klinische Methoden der Blutgerinnungsanalyse, Thieme-Verlag 1959) bestimmt. Die Angaben in % der Norm beziehen sich auf Humancitratplasma.

Der Fibrinogengehalt wurde nach der Methode von Blombäck [Blombäck, Arkiv Kemi 10, 415-442 (1956)] bestimmt [mit Modifikation von Jacobson, Scand. J. Clin. and Lab. Invest. Suppl. 14 (1955), 1-102.]

| Ansatz | Fibrinogen mg % | Faktorengehalt in % bezüglich Normalhumanplasma | | | |
|--------|-----------------|------|------|--------|------|
|        |                 | II | V | VII/X | X |
| 1 | 387 | 6.9 | 335 | 9 | 16 |
| 2 | 355 | 7.3 | 300 | 11 | 20.5 |
| stabil antikoaguliertes Humanplasma | 300 | 10.5 | 115 | 15.5 | 13.9 |

Patentansprüche

1.    Kontrollplasma nicht menschlicher Herkunft zur Ueberwachung der oralen Antikoagulantientherapie beim Menschen,
hergestellt aus Blut von stabil antikoagulierten Säugetieren.

2.    Kontrollplasma nach Anspruch 1 hergestellt aus Blut
von Schafen.

3.    Kontrollplasma nach Anspruch 1 oder Anspruch 2, dadurch gekennzeichnet, dass das Verhältnis seiner Faktor V-
Aktivität zu derjenigen eines menschlichen Plasmas grösser
als 2 ist.

4.    Kontrollplasma nach Anspruch 3, dadurch gekennzeichnet, dass das Verhältnis seiner Faktor V-Aktivität zu derjenigen eines menschlichen Plasmas 2 bis 5 ist.

5.    Kontrollplasma nach einem der Ansprüche 1 bis 4 enthaltend ein Mittel zur Verhinderung der spontanen Blutgerinnung.

6.    Kontrollplasma nach Anspruch 5 enhaltend Trinatriumcitrat.

7.    Kontrollplasma nach einem der Ansprüche 1 bis 6 enthaltend einen Puffer zum Einstellen eines physiologischen
pH-Wertes.
8.    Kontrollplasma nach Anspruch 7 enthaltend N-2-Hydro-
xyäthylpiperazin-N'-aethansulfonsäure.

9.    Kontrollplasma nach einem der Ansprüche 1 bis 8, in
lyophilisierter Form.

10.    Verfahren zur Herstellung eines Kontrollplasmas zur Ueberwachung der oralen Antikoagulantientherapie beim Menschen, dadurch gekennzeichnet, dass man Säugetiere mit einem Antikoagulans behandelt, sie mittels Ueberwachung von Quickwert- oder Thromboplastinzeitquotient auf einen Wert im therapeutischen Bereich einstellt, das Plasma nach Blutentnahme in einem Mittel, das die spontane Blutgerinnung verhindert, durch Zentrifugation gewinnt und dem erhaltenen Plasma einen Puffer zum Einstellen eines physiologischen pH-Wertes zusetzt.

11.    Verfahren nach Anspruch 10, dadurch gekennzeichnet, dass als Säugetiere Schafe eingesetzt werden.

12.    Verfahren nach Anspruch 10 oder Anspruch 11, dadurch gekennzeichnet, dass das Mittel zur Verhinderung der spontanen Blutgerinnung Trinatriumcitrat ist.

13.    Verfahren nach einem der Ansprüche 10 bis 12, dadurch gekennzeichnet, dass der Puffer zum Einstellen eines physiologischen pH-Wertes N-2-Hydroxyäthylpiperazin-N'-äthansulfonsäure ist.

14.    Verfahren nach einem der Ansprüche 10 bis 13, dadurch gekennzeichnet, dass nach Zusatz des Puffers das Plasma erneut zentrifugiert wird.

15.    Verfahren nach einem der Ansprüche 10 bis 14, dadurch gekennzeichnet, dass das erhaltene Kontrollplasma lyophilisiert wird.

- 12 -

0005243

16. Reagenziengarnitur zur Ueberwachung der Antikoagulantientherapie beim Menschen enthaltend

a) in einem ersten Behälter ein Kontrollplasma gemäss einem der Ansprüche 1 bis 9

b) gegebenenfalls in einem zweiten Behälter ein normales menschliches Kontrollplasma

c) gegebenenfalls in einem dritten Behälter ein Thromboplastinreagens

d) gegebenenfalls in einem vierten Behälter ein Mittel zur Verhinderung der spontanen Blutgerinnung.

0005243

17.    Verwendung eines Kontrollplasmas gemäss einem der
Ansprüche 1 bis 9 zur Ueberwachung der Antikoagulantientherapie beim Menschen.


*** *